(19) Europäisches Patentamt
European Patent Office
Office européen des brevets

(11) Veröffentlichungsnummer: **0 519 433 A1**

(12) **EUROPÄISCHE PATENTANMELDUNG**

(21) Anmeldenummer: **92110244.8**

(22) Anmeldetag: **17.06.92**

(51) Int. Cl.[5]: **C07D 295/20**, C07D 401/12, C07D 401/14, C07D 413/14, C07D 417/14, A61K 31/535, C07K 5/06

(30) Priorität: **21.06.91 DE 4120510**

(43) Veröffentlichungstag der Anmeldung: **23.12.92 Patentblatt 92/52**

(84) Benannte Vertragsstaaten: **AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Heitsch, Holger, Dr.**
**Martin-Wohmann-Strasse 27**
**W-6238 Hofheim am Taunus(DE)**
Erfinder: **Henning, Rainer, Dr.**
**Im Höhlchen 16**
**W-6234 Hattersheim/M.(DE)**
Erfinder: **Urbach, Hansjörg, Dr.**
**Le Lavandoustrasse 41**
**W-6242 Kronberg/Ts.(DE)**
Erfinder: **Ruppert, Dieter, Dr.**
**Schreyerstrasse 30**
**W-6242 Kronberg/Ts.(DE)**
Erfinder: **Linz, Wolfgang, Dr.**
**Huxelrebenweg 54**
**W-6500 Mainz 42(DE)**

(54) **Verbindungen mit reninhemmenden Eigenschaften, Verfahren zu ihrer Herstellung und ihre Verwendung.**

(57) Verbindungen der Formel (I)

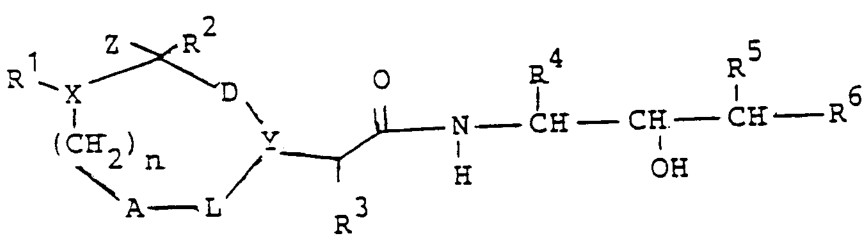

(I)

| | |
|---|---|
| X, Y | z.B. N |
| $R^1$,$R^2$,$R^3$ | z.B. Wasserstoff |
| A,L | z.B. $CH_2$ oder CO |
| Z | z.B. Wasserstoff |
| $R^4$ | z.B. Cyclohexylmethyl |
| $R^5$ | z.B. Hydroxy |
| $R^6$ | z.B. |

EP 0 519 433 A1

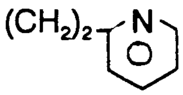

n z.B. Null oder 1

sind wirksame Renin-inhibierende Verbindungen, die eine hohe in-vitro- und vivo-Aktivität aufweisen.

Aus EP-A 370 454, EP-A 373 497, EP-A 394 853 und EP-A 417 698 sind heterocyclisch substituierte Aminodiolderivate mit renininhibitorischer Wirkung bekannt. In EP-A 365 992 werden N-terminal konformativ fixierte Alkylalkohol- und Statin-Derivate beschrieben.

Es wurde nun gefunden, daß Verbindungen, die eine vorzugsweise gesättigte, heterocyclische Gruppierung am N-Terminus und einen heterocyclischen Substituenten am C-Terminus des Moleküls aufweisen, außergewöhnlich wirksame und hochspezifische Reninhemmer sind, die eine wesentlich verbesserte Wirkung in vivo und eine erheblich gesteigerte Resorption aufweisen.

Die Erfindung betrifft daher eine Verbindung der Formel (I)

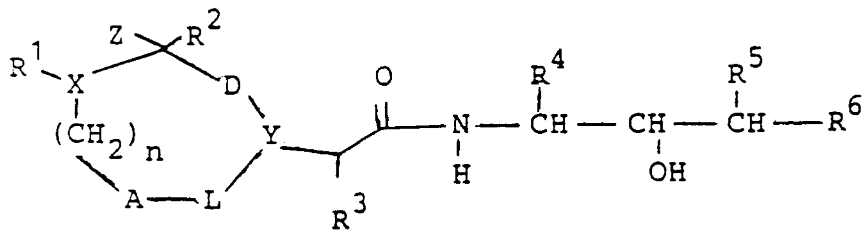

(I)

in welcher bedeuten:

X N, O oder CH;

$R^1$ nicht vorhanden, Wasserstoff, eine N-Schutzgruppe, Aryl, heterocyclisch oder $R^7$-Q mit

1) $R^7$ gleich kurzkettiges Alkyl, Amino, Alkylamino, Dialkylamino, (Alkoxyalkyl)(alkyl)amino, (Alkoxyalkoxyalkyl)(alkyl)amino, Aryl, Arylalkyl, Aminoalkyl, N-geschütztes Aminoalkyl, Alkoxy, substituiertes kurzkettiges Alkyl mit einem Substituenten aus der Gruppe: Alkoxy, Thioalkoxy, Halogen, Alkylamino, N-geschütztes (Alkyl)amino und Dialkylamino,

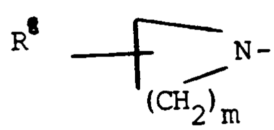

mit m gleich 1-5 und $R^8$ Wasserstoff, Hydroxy, Alkoxy, Thioalkoxy, Alkoxyalkoxy, Polyalkoxy, Amino, N-geschütztes Amino, Alkylamino, N-geschütztes Alkylamino oder Dialkylamino,

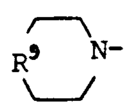

mit $R^9$ gleich O, S, $SO_2$, O=C oder $R^{10}N$ mit $R^{10}$ gleich Wasserstoff, kurzkettiges Alkyl oder eine N-Schutzgruppe,

2) Q ist C=O oder $CH_2$, wobei X gleich N ist, wenn $R^1$ eine N-Schutzgruppe ist,

$R^{11}$ $S(O)_2$ mit $R^{11}$ gleich Amino, Alkylamino, Dialkylamino, kurzkettiges Alkyl, Halogenalkyl, Aryl, p-Biphenyl, heterocyclisch oder $(R^{12})_2P(O)$ mit $R^{12}$ gleich Alkoxy, Alkylamino oder Dialkylamino,

A und L sind unabhängig voneinander entweder nicht vorhanden, C=O, $SO_2$ oder $CH_2$;

Y N oder CH;

$R^2$ Wasserstoff, kurzkettiges Alkyl, Cycloalkylalkyl, $-CH_2-R^{13}-(CH_2)_q-R^{14}$, wobei q gleich 0, 1 oder 2 ist;

$R^{13}$ nicht vorhanden oder O, NH oder S bei q gleich 1 oder 2 ist;

$R^{14}$ für Alkyl oder einen Heterocyclus steht;

Z Wasserstoff oder $-R^{15}C(O)R^{16}$, $-R^{15}S(O)_2R^{16}$ oder $-R^{15}-C(S)R^{16}$, wobei

$R^{15}$ gleich NH, $-N(R^{17})-$ mit $R^{17}$ gleich kurzkettiges Alkyl oder Benzyl, $CH_2$ und

$R^{16}$ ist Alkoxy, Benzyloxy, Alkylamino, Dialkylamino, Aryl oder heterocyclisch;

$R^3$ Wasserstoff, kurzkettiges Alkyl, kurzkettiges Alkenyl, Cycloalkylalkyl, Cycloalkenylalkyl, Alkoxyalkyl, Thioalkoxyalkyl, (Alkoxyalkoxy)alkyl, (Polyalkoxy)alkyl, Arylalkyl oder heterocyclisch substituiertes Alkyl;

n 0 oder 1

$R^4$ Wasserstoff, ($C_1$-$C_{10}$)-Alkyl, ($C_3$-$C_6$)-Cycloalkyl, ($C_3$-$C_8$)-Cycloalkyl-($C_1$-$C_4$)-alkyl, ($C_6$-$C_{12}$)-Aryl oder ($C_6$-$C_{12}$)-Aryl-($C_1$-$C_4$)-alkyl

$R^5$ Wasserstoff, ($C_1$-$C_{10}$)-Alkyl, ($C_6$-$C_{12}$)-Aryl, ($C_6$-$C_{12}$)-Aryl-($C_1$-$C_4$)-alkyl, Hydroxy oder Amino;

$R^6$ einen Rest der Formel (II)

$(CH_2)_s$-$CHR^{18}$-Het (II)

wobei

$R^{18}$ für Wasserstoff, ($C_1$-$C_7$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht, und Het für einen 5- bis 7-gliedrigen heterocyclischen Ring steht, der benzanneliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Reihe ($C_1$-$C_4$)-Alkyl, Allyl, ($C_1$-$C_4$)-Alkoxy, Hydroxy, Halogen, Amino, Mono- oder Di-($C_1$-$C_4$)-alkylamino und $CF_3$ substituiert sein kann; und

s 0, 1, 2, 3 oder 4 bedeutet,

sowie deren physiologisch verträglichen Salze.

Bevorzugt wird dabei eine heterocyclische Verbindung der Formel (I) oder ein Salz dieser Verbindung, bei der Symbole folgende Bedeutungen haben:

$R^4$ Isobutyl, Benzyl oder Cyclohexylmethyl;

$R^5$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy;

$R^6$ einen Rest der Formel (II), worin $R^{18}$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht,

s 0, 1 oder 2.

Ebenfalls bevorzugt sind folgende Bedeutungen für die Symbole:

X N oder CH,

$R^1$ nicht vorhanden, Wasserstoff, Benzyl, 2-Pyridyl, Piperazin-1-yl, (4-OMe)phenyl, tert.-Butyloxycarbonyl, Isobutyl, Benzyloxycarbonyl, $Me_2NC(O)$-, 4-Methyl-piperazin-1-yl-carbonyl, Morpholin-1-yl-carbonyl, p-Toluolsulfonyl oder 4-Methyl-piperazin-1-yl-sulfonyl;

A und L unabhängig voneinander entweder nicht vorhanden oder $CH_2$,

D C = O oder $CH_2$;

Y gleich N

$R^2$ Wasserstoff oder Benzyl;

Z Wasserstoff, BOC-NH, CbZ-NH, p-Toluolsulfonylamino, 4-Methylpiperazin-1-yl-sulfonylamino, 4-Methylpiperazin-1-yl, N-Methyl-N-(4-methyl-piperazin-1-yl-sulfonyl)-amino;

$R^3$ Wasserstoff, Methyl, n-Butyl, 4-Imidazomethyl, 4-Thiazolylmethyl oder N-BOC-4-(imidazomethyl); und

n Null.

Die Erfindung betrifft insbesondere eine heterocylische Verbindung der Formel (I), bei der die Symbole folgende Bedeutungen haben:

$R^4$ Isobutyl, Benzyl oder Cyclohexylmethyl;

$R^5$ Wasserstoff oder Hydroxy;

$R^6$ einen Rest der Formel (II), worin

$R^{18}$ für Wasserstoff oder Fluor steht,

Het für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Alkyl, Fluor, Chlor, Brom, $CF_3$ und Methoxy substituiert sein können, und

s 0, 1 oder 2;

und die übrigen Reste und Variablen wie oben definiert sind, sowie deren physiologisch verträglichen Salze.

Die Chiralitätszentren in den Verbindungen der Formel (I) können die R, oder S-Konfiguration aufweisen.

Der Terminus "N-Schutzgruppe" bezeichnet solche Gruppen, die geeignet sind, Stickstoff-Atome während synthetischer Verfahren vor unerwünschten Reaktionen zu schützen, den Angriff von Exopeptidasen in den Endprodukten zu verhindern oder zur Erhöhung der Löslichkeit beitragen. Der Begriff bezeichnet Gruppen wie z.B. Acyl-, Acetyl-, Pivaloyl-, t-Butylacetyl-, Trichlorethoxycarbonyl-, t-Butyloxycarbonyl-(BOC),

Benzyloxycarbonyl-(CbZ)- oder Benzoyl-Gruppen oder einen L- oder D-Aminoacyl-Rest, der selbst wiederum in gleicher Weise N-geschützt ist.

Der Terminus "kurzkettiges Alkyl" steht für unverzweigte oder verzweigte Alkylreste mit 1 bis 7 C-Atomen wie z.B. Methyl, n-Propyl, iso-Propyl, n-Butyl, iso-Butyl, sec-Butyl, n-Pentyl, 1-Methylbutyl, 2,2-Dimethylbutyl, 2-Methylpentyl, 2,2-Dimethylpropyl oder n-Hexyl.

Der Terminus "kurzkettiges Alkenyl" steht für kurzkettige Alkylreste, die mindestens eine C-C-Doppelbindung enthalten.

Der Terminus "Alkylamino" bezeichnet -NH, verknüpft mit einem kurzkettigen Alkylrest.

Der Terminus "Aminoalkyl" bezeichnet $-NH_2$, verknüpft mit einem kurzkettigen Alkylrest.

Der Terminus "Cycloalkyl" steht für einen aliphatischen Ring aus 3-7 C-Atomen.

Der Terminus "Aryl" bezeichnet mono- oder bicyclischer Kohlenstoff-Ringsysteme mit einem oder mehreren aromatischen Ringen wie Phenyl, Naphthyl-Tetrahydronaphthyl, Indanyl und dergleichen. Der Begriff "Aryl" steht ebenso für heterocyclische, aromatische Ringsysteme. Aryl-Reste können dabei unsubstituiert oder substituiert sein durch 1, 2 oder 3 Substituenten, die unabhängig voneinander kurzkettiges Alkyl, Halogenalkyl, Alkoxy, Thioalkoxy, Amino, Alkylamino, Hydroxy, Halogen, Mercapto, Nitro, Carboxyaldehyd, Carboxy und Carboxamide sein können.

Der Terminus "Polyalkoxy" steht für $-OR^{19}$ mit $R^{19}$ gleich einer verzweigten oder unverzweigten Alkylkette mit 1-5 $C_w$-O-$C_x$-Bindungen, bei der w und x unabhängig voneinander 1-3 sein können.

Der Terminus "heterocyclisches Alkyl" steht für einen mit einem kurzkettigen Alkylrest substituierten Heterocyclus wie z.B. Imidazolylmethyl oder Thiazolylmethyl.

Halogen ist Fluor, Chlor, Brom oder Jod.

Unter $(C_1-C_{10})$-Alkyl ist vorzugsweise ein Alkylrest oder ein entsprechend ungesättigter Rest mit 1-10 C-Atomen zu verstehen. Der Rest kann geradkettig oder verzweigt sein. Entsprechendes gilt für davon abgeleitete Reste, wie z.B. Alkoxy, Alkylthio, Alkylamino, Alkanoyl und Aralkyl.

Unter $(C_3-C_8)$-Cycloalkyl werden vorzugsweise Cyclopropyl, Cyclobutyl, Cyclopentyl, Cyclohexyl, Cycloheptyl und Cyclooctyl verstanden. Falls diese Cyclen mehr als einen Substituenten tragen, so können diese sowohl cis als trans zueinander stehen.

Unter einem heteroaromatischen Rest versteht man insbesondere einen Rest, der einen sechsgliedrigen, vom Benzol abgeleiteten aromatischen Ring enthält, worin eine oder mehrere CH-Gruppen durch N ersetzt, oder der einen fünfgliedrigen, vom Benzol abgeleiteten aromatischen Ring enthält, worin eine oder zwei CH-Gruppen durch O, S, NH oder N ersetzt sind und worin gegebenenfalls weitere CH-Gruppen durch N ersetzt sind. Dieser Heteroaryl-Rest ist vorzugsweise monocyclisch oder bicyclisch, wobei er im letzteren Fall mit einem Benzol oder einem wie vorstehend definierten Fünf- oder Sechsring anneliert ist. Entsprechendes gilt für davon abgeleitete Reste, wie die heteroaromatischen-aliphatischen Reste Heteroaralkyl, Heteroaroyl oder für Heteroaryloxy.

Ein Rest Het im Sinne vorstehender Definition ist beispielsweise ein Heteroarylrest wie Pyrrolyl, Furyl, Thienyl, Imidazolyl, Pyrazolyl, Oxyzolyl, Thiazolyl, Pyridyl, Pyrazinyl, Pyrimidinyl, Indolyl, Chinolyl, Isochinolyl, Chinoxalinyl, β-Carbolinyl oder ein benzanneliertes, cyclopenta-, cyclohexa- oder cyclohepta-anneliertes Derivat dieser Reste. Dieser Heterocyclus kann an einem Stickstoffatom durch Oxido, $(C_1-C_6)$-Alkyl, z.B. Methyl oder Ethyl, Phenyl oder Phenyl-$(C_1-C_4)$-alkyl, z.B. Benzyl und/oder an einem oder mehreren C-Atomen durch $(C_1-C_4)$-Alkyl, z.B. Methyl, Phenyl, Phenyl-$(C_1-C_4)$-alkyl, z.B. Benzyl, Halogen, z.B. Chlor, Hydroxy, $(C_1-C_4)$-Alkoxy, z.B. Methoxy, Phenyl-$(C_1-C_4)$-alkoxy, z.B. Benzyloxy oder Oxo bis zu trisubstituiert und teilweise gesättigt sein.

Beispiele sind: 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-pyridino, 2-Pyrazinyl, 2-, 4- oder 5-Pyrimidyl, 2-, 3- oder 5-Indolyl, substituiertes 2-Indolyl, z.B. 1-Methyl-, 5-Methyl-, 5-Methoxy, 5-Benzyloxy, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[c]indol-2-yl oder β-Carbolin-3-yl.

Teilhydrierte oder vollständig hydrierte, heterocyclische Ring sind beispielsweise Dihydropyrdinyl, Pyrrolidinyl, z.B. 2-, 3- oder 4-N-Methylpyrrolidinyl, Piperidinyl, Piperazinyl, Morpholino, Thiomorpholino, Tetrahydrothiophenyl.

Unter den verwendeten Terminus "gegebenenfalls substituiert" versteht man, daß der betreffende Rest mit einem, zwei oder drei, vorzugsweise einem oder zwei gleichen oder verschiedenen Resten aus der Reihe $(C_1-C_6)$-Alkyl, $(C_1-C_6)$-Alkoxy, Hydroxy, Halogen, $(C_1-C_6)$-Alkanoyl, $CF_3$, Amino, $(C_1-C_6)$-Alkylamino, Di-$(C_1-C_6)$-alkylamino, Carbamoyl, $(C_1-C_6)$-Alkoxycarbonyl und Sulfamoyl substituiert sein kann.

Unter Salzen von Verbindungen der Formel (I) sind insbesondere pharmazeutisch verwendbare und

nicht-toxische Salze zu verstehen.

Solche Salze werden beispielsweise von Verbindungen der Formel (I), welche saure Gruppen, z.B. Carboxy enthalten, mit Alkali- oder Erdalkalimetallen gebildet, wie Na, K, Mg, und Ca, sowie mit physiologisch verträglichen organischen Aminen, wie z.B. Triethylamin.

Verbindungen der Formel (I), welche basische Gruppen, z.B. eine Aminogruppe oder eine Guanidinogruppe enthalten, bilden Salzen mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure ooder $\beta$-Toluolsulfonsäure.

Die Erfindung betrifft weiterhin ein Verfahren zur Herstellung von Verbindungen der Formel (I), das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe (n) abspaltet und die so erhaltene Verbindung gegebenenfalls in ihr physiologisch verträgliches Salz überführt.

Fragmente einer Verbindung der Formel (I) mit einer endständigen Carboxylgruppe besitzen die nachstehende Formel (III)

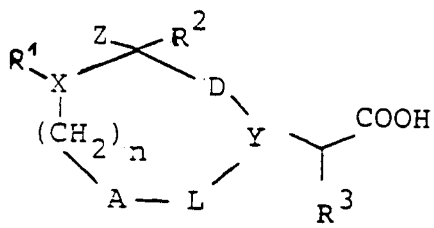

(III)

Fragmente einer Verbindung der Formel (I) mit einer endständigen Aminogruppe besitzen die nachstehende Formel (IV)

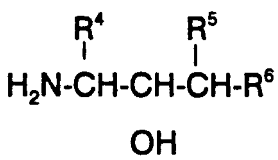

(IV)

Methoden, die zur Herstellung einer Amidbindung geeignet sind, werden z.B. in Houben-Weyl, Methoden der organischen Chemie, Band 15/2, Bodanszky et al., Peptide Synthesis, 2nd ed. (Wiley Sons; New York 1976) oder Grass, Meilenhofer, The Peptides, Analysis, Synthesis, Biology (Academic Press, New York 1979) beschrieben. Vorzugsweise werden die folgenden Methoden herangezogen: Aktivestermethode mit N-Hydroxy-succinimid oder 1-Hydroxybenzotriazol als Esterkomponente, Kupplung mit einem Carbodiimid wie Dicyclohexylcarbodiimid oder mit Propanphosphonsäureanhydrid oder Methylethylphosphinsäureanhydrid und die Gemischt-Anhydrid-Methode mit Pivaloylchlorid (M. Zaoral, Coll. Chem. Commun., 1962, 27, 1273). Man führt die Reaktionen vorzugsweise in einem inerten Lösungsmittel oder Lösungsmittelgemischen bei einer Temperatur zwischen -20 °C und dem Siedepunkt des Reaktionsgemisches durch.

Die Herstellung der Carbonsäuren der Formel (III) erfolgt z.B. nach EP-A 365 992. Die Herstellung der als Ausgangsverbindungen verwendeten, optisch aktiven Amine der Formel (IV), worin $R^4$, $R^5$ und $R^6$ wie oben definiert sind, erfolgt ausgehend von optisch aktiven $\alpha$-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppen Aminoalkohole der Formel (IV) ergibt. Bei $R^5$ = OH dient ebenfalls ein N-geschützter Aminosäurealdehyd als Ausgangsmaterial, der z.B. durch aldol-analoge Addition von ungesättigten Verbindungen, Einführung geeigneter Schutzgruppen und anschließende Epoxidierung in die benötigten Zwischenprodukte umgewandelt wird. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt mittels HPCL; die Enantiomerenreinheit kann in bekannter Weise durch Überführung in Mosher-Derivate überprüft werden (H.S. Mosher et al., J. Org. Chem. 34 (1969) 2543).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butylcarbonyl- und Benzyloxycarbonyl-Schutzgruppen) erfolgt in einem gegenüber Basen inertem Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die zur Herstellung von Verbindungen der Formel (I) erforderlichen Vor- und Nachoperationen wie Einführung und Abspaltung von Schutzgruppen sind literaturbekannt und sind z.B. in T.W. Greene, "Protective Groups in Organic Synthesis", beschrieben. Salze von Verbindungen der Formel (I) mit salzbildenden Gruppen werden in an sich bekannter Weise hergestellt, indem man z.B. eine Verbindung der Formel (I) mit einer basischen Gruppe mit einer stöchiometrischen Menge einer geeigneten Säure umsetzt.

Stereoisomerengemische, insbesondere Diastereomerengemischen, die bei Verwendung racemischer Carbonsäurederivate anfallen, können in an sich bekannter Weise durch fraktionierte Kristallisation oder durch Chromatographie getrennt werden.

Die erfindungsgemäßen Verbindungen der Formel (I) weisen enzymhemmende Eigenschaften auf; insbesondere hemmen sie die Wirkung des natürlichen Enzyms Renin. Renin ist ein proteolytisches Enzym aus der Klasse der Aspartyl-Proteasen, welches als Folge verschiedener Stimuli (Volumendepletion, Natriummangel, $\beta$-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration diese aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

1. Testprinzip

Z.B. Humanplasma, welches sowohl Renin als auch Angiotensinogen enthält, wird bei 37 °C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotenin-Freisetzung wird durch Renin-Inhibitoren gehemmt.

2. Gewinnung des Plasmas

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3500 Upm, 0-4 °C, 15 min; wiederholen, falls erforderlich) wird das Plasma vorsichtig abpipettiert und in geeignete Portionen bei -30 °C eingefroren. Für den Test werden nur Plasmen mit ausreichend hoher Reninaktivität verwendet. Plasmen mit niedriger Reninaktivität werden durch eine Kältebehandlung (-4 °C, 3 Tage) aktiviert (Prorenin → Renin).

3. Durchführung des Tests

Angiotensin I wird mit dem Renin-Maia®-Kit (Sereno Diagnostics S.A., Coinsins, Schweiz) bestimmt. Die Inkubation des Plasmas wird nach der dort angegebenen Anleitung durchgeführt.

| | |
|---|---|
| Inkubationsansatz: | 1000 µl Plasma (bei 0-4 °C aufgetaut) |
| | 100 µl Phosphatpuffer (pH 7,4) Zusatz von $10^{-4}$ M Ramiprilat) |
| | 10 µl PMSF-Lösung |
| | 10 µl 0,1 % Genapol PFIC |
| | 12 µl DMSO bzw. Testpräparat |

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansäte werden in Eis gemischt und für 1 Stunde zur Inkubation in ein Wasserbad (37 ° C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentration der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockenes eingefroren und bei ca. -25 ° C für die Angiotensin I-Bestimmung aufbewahrt (Mittelwert aus drei Einzelproben). Angiotensin I-Radioimmunoassay (RIA)

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehatl des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel (I) zeigen in dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vitro-Test von Renin-Hemmern Primaten (Marmosets, Rhesus-Affen) herangezogen. Primaten-Renin und Human-Renin sind in ihre Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel (I) können als Antihypertensiva, sowie zur Behandlung der Herzinsuffizienz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirkung, die die erfindungsgemäßen Verbindungen insbesondere zur Therapie und Prophylaxe von durch Injektion mit HIV bedingten Erkrankungen qualifiziert. Die erfindungsgemäßen Verbindungen der allgemeinen Formel (I) zeigen in verwendeten in-vitro-Tests Hemmwirkungen bei Konzentrationen von etwa $10^{-4}$ bis $10^{-8}$ Mol/l.

Zum Gegenstand der Erfindung gehört weiterhin die Verwendung von Verbindungen der Formel (I) zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel (I) zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutische Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Verzeichnis der verwendeten Abkürzungen:

| | |
|---|---|
| Boc | tert.-Butoxycarbonyl |
| BuLi | n-Butyl-Lithium |
| DCC | Dicyclohexylcarbondiimid |
| DCI | Desorption Chemical Ionisation |
| DNP | 2,4-Dinitrophenyl |
| DME | Dimethoxyethan |
| DMF | Dimethylformamid |
| EE | Essigsäureethylester |
| FAB | Fast atom bombardment |
| h | Stunde |
| HOBt | 1-Hydroxybenzotriazol |
| M | Molekularpeak |
| MS | Massenspektrum |
| min | Minuten |
| NEM | N-Ethylmorpholin |
| RT | Raumtemperatur |
| THF | Tetrahydrofuran |
| Trt | Triphenylmethyl |

Die folgenden Beispiele illustrieren die Erfindung.

Beispiel 1

N-[2S-[(3R-Benzyl-4-N-(morpholin-1-yl-carbonyl)-piperazin-1-yl)]-hexanoyl]-(2S,3R 4S)-1-cyclohexyl-3,4-dihydroxy-6(2-pyridyl)-2-hexylamin

a) (2S, 3R, 4S)-2-(tert-Butyloxycarbonyl)amino-1-cyclohexyl-3,4-dihydroxy-6(2-pyridyl)-hexan

186 mg 2-Picolin in 20 ml THF werden bei -78° C mit 2,8 ml n-Butyllithium versetzt. Nach Aufwärmen auf Raumtemperatur wird 30 Min. gerührt, dann auf -40° C abgekühlt. 2 mmol (2RS, 3R, 4S)-3-tert.-Butyldimethyl-silyloxy-4-(tert.-butyloxycarbonyl)amino-5-cyclohexyl-1,2-oxopentan (bekannt aus EP-A 189 203, Beispiel 6) werden zugegeben (gelöst in 10 ml THF). Nach 10 h Rühren bei Raumtemperatur wird mit Wasser verdünnt und mit Methyl-tert.-butylether extrahiert. Das Rohprodukt wird nach Einengen in THF gelost und mit 10 ml einer 1 M Lösung von Tetrabutylammoniumfluorid in THF 1 h bei 0° C gerührt. Nach Verdünnen mit Wasser, Extrahieren mit EE und Einengen erhält man 300 mg des (2S, 3R, 4S)-Isomeren MS(FAB) 393 (M + H) und 240 mg des (2S, 3S, 4S)-Isomeren MS(FAB) 393 (M + H) nach chromatographischer Trennung an Kieselgel.

b) N-[2S-(3R-Benzyl-4-N-(morpholin-1-yl-carbonyl)-piperazin-1-yl)-hexanoyl]-(2S, 3R,4S)-1-cyclohexyl-3,4-dihydroxy-6(2-pyridyl)-2-hexylamin

200 mg (2S, 3R,4S)-2-(tert.-Butyloxycarbonyl)amino-1-cyclohexyl-3,4-dihydroxy-6(2-pyridyl)-hexan (Verbindung aus Beispiel 1a) werden mit 3ml Trifluoressigsäure in 3 ml abs. Methylenchlorid für 2 h bei Raumtemperatur gerührt. Der nach Einengung resultierende Rückstand wird in 1 M $NaHCO_3$-Lösung aufgenommen, das Gemisch dreimal mit EE extrahiert und die EE-Phasen über $Na_2SO_4$ getrocknet. Nach Einengung wird der Rückstand in 2ml abs. DMF gelöst, 205,8 mg 2-(3R-Benzyl-4-N-(morpholin-1-yl-carbonyl)-piperazin-1-yl)-hexansäure (bekannt aus EP-A 365 992, Beispiel 38), 104,2 mg N,N'-Dicyclohexylcarbodiimid und 103,4 mg 1-Hydroxybenzotriazol hinzugegeben, der pH der Lösung mit N-Ethylmorpholin auf 9 eingestellt und 48 h bei Raumtemperatur gerührt. Nach Filtration wird mit EE verdünnt und je einmal mit gesättigter $NaHCO_3$-Lösung, Wasser und gesättigter Kochsalzlösung gewaschen, über $Na_2SO_4$ getrocknet und eingeengt. Chromatographie an Kieselgel (mit einem $CH_2Cl_2$/MeOH-Laufmittelgemisch) ergibt 93,7 mg der Titelverbindung.
MS(FAB): 678 (M + H)

Beispiel 2

N-[2S-(3R-Benzyl-4-N-tert.-butyloxycarbonyl-2-keto-piperazin-1-yl)-hexanoyl]-(2S,3R,4S)-1-cyclohexyl-3,4-dihydroxy-6(2-pyridyl)-2-hexylamin

Die Titelverbindung wird nach dem in Beispiel 1b beschriebenen Verfahren aus 2-(3R-Benzyl-4-N-tert.-butyloxycarbonyl-2-keto-piperazin-1-yl)-hexansäure (bekannt aus EP-A 365 992, Beispiel 43) und der Verbindung aus Beispiel 1a hergestellt.
MS(FAB): 679 (M + H)

Beispiel 3

N-[3-(4-Imidazolyl)-2S-(3R-benzyl-4-N-tert.-butyloxycarbonyl-2-keto-piperazin-1-yl)propionyl]-(2S,3R,4S)-1-cyclohexyl-3,4-dihydroxy-6(2-pyridyl)-2-hexylamin

Die Titelverbindung resultiert aus dem Umsatz von 3-(4-Imidazolyl)-2S-(3R-benzyl-4-N-tert.-butyloxycarbonyl-2-keto-piperazin-1-yl)-propionsäure (hergestellt nach EP-A 365 992, Beispiel 45) und der Verbindung aus Beispiel 1a nach dem in Beispiel 1b angeführten Verfahren.
MS(FAB): 703 (M + H)

Beispiel 4

N-[2S-(3-((Benzyloxycarbonyl)amino)-3-phenylmethyl-2-oxo-1-pyrrolidinyl)-3-(4-imidazolyl)propionyl]-(2S,3R,4S)-1-cyclohexyl-3,4-dihydroxy-6(2-pyridyl)-2-hexylamin

Die Titelverbindung wird nach dem in Beispiel 1b beschriebenen Verfahren aus (2S)-((3R,3S)-(Benzyloxycarbonyl)amino)-3-phenylmethyl-2-oxo-1-pyrrolidinyl)-3-(4-imidazolyl)propionsäure (bekannt aus EP-A 365 992, Beispiel 62) und der Verbindung aus Beispiel 1a hergestellt.
MS(FAB): 737 (M + H)

Beispiel 5

N-[2S-(3S-(Benzyloxycarbonyl)amino)-3-phenylmethyl-2-oxo-1-piperidinyl)-3-(4-imidazolyl)propionyl]-(2S,3R,4S)-1-cyclohexyl-3,4-dihydroxy-6(2-pyridyl)-2-hexylamin

Diese Verbindung wird aus (2S)-2-((1-tert.-Butyloxycarbonyl)-1-imidazol-4-yl-methyl)-3-((3S)(-(benzyloxycarbonyl)amino)-3-phenylmethyl-2-oxo-1-piperidinyl)essigsäure-Lithiumsalz (bekannt aus EP-A 365 992, Beispiel 84) und der Verbindung aus Beispiel 1a nach dem in Beispiel 1b beschriebenen Verfahren und sich anschließender Schutzgruppenabspaltung in THF (1 ml), Essigsäure (3 ml) und Wasser (1 ml) dargestellt.
MS(FAB): 753 (M + H)

Beispiel 6

N-[3-(4-Thiazolyl)-2-(3R-benzyl-4-N-morpholin-1-yl-carbonyl-2-keto-piperazin-1-yl)propionyl]-(2S,3R,4S)-1-cyclohexyl-3,4-dihydroxy-6(2-pyridyl)-2-hexylamin

Die Titelverbindung wird aus 3-(4-Thiazolyl)-2-(3R-benzyl-4-N-morpholin-1-yl-carbonyl-2-keto-piperazin-1-yl)-propionsäure (bekannt aus EP-A 365 991, Beispiel 129) und der Verbindung aus Beispiel 1a nach dem in Beispiel 1b angeführten Verfahren hergestellt.
MS(FAB): 733 (M + H)

Beispiel 7

N-[3-(4-Thiazolyl)-2-(3R-benzyl-4-N-p-toluolsulfonyl-2-keto-piperazin-1-yl)-propionyl]-(2S,3R,4S)-1-cyclohexyl-3,4-dihydroxy-6(2-pyridyl)-2-hexylamin

Die Titelverbindung wird aus 3-(4-Thiazolyl)-2-(3R-benzyl-4-N-p-toluolsulfonyl-2-keto-piperazin-1-yl)-propionsäure (bekannt aus EP-A 365 992, Beispiel 130) und der Verbindung aus Beispiel 1a nach dem in Beispiel 1b angegebenen Verfahren hergestellt.

MS(FAB): 774 (M + H)

Beispiel 8

N-[3-(4-Thiazolyl)-2-(3R-benzyl-4-N-(N-methylpiperazyl)-sulfonyl-2-keto-piperazin-1-yl)-propionyl]-(2S,3R,4S)-1-cyclohexyl-3,4-dihydroxy-6(2-pyridyl)-2-hexylamin

Die Titelverbindung wird aus 3-(4-Thiazolyl)-2-(3R-benzyl-4-N-(N-methylpiperazyl)sulfonyl-2-keto-piperazin-1-yl)propionsäure (bekannt aus EP-A 365 992, Beispiel 141) und der Verbindung aus Beispiel 1a nach dem Verfahren des Beispiels 1b dargestellt.
MS(FAB): 782 (M + H)

Beispiel 9

N-[3-(4-Imidazolyl)-2-(3R-benzyl-4-N-(N-methylpiperazyl)-sulfonyl-2-keto-piperazin-1-yl)-propionyl]-(2S,3R,4S)-1-cyclohexyl-3,4-dihydroxy-6(2-pyridyl)-2-hexylamin

Die Titelverbindung wird aus 3-(1N-tert.-Butyloxycarbonyl-4-imidazolyl)-2(3R-benzyl-4-N-(N-methylpiperazyl)-sulfonyl-2-keto-piperazin-1-yl)-propionsäure (bekannt aus EP-A 365 992, Beispiel 1 und 2) und der Verbindung aus Beispiel 1a nach dem in Beispiel 1b angegebenen Verfahren mit anschließender Schutzgruppenabspaltung mit $CF_3COOH$ in $CH_2CH_2$ und extraktiver Aufarbeitung hergestellt.
MS(FAB): 765 (M + H)

Beispiel 10

N-[(2S)-((35)-(N-Methylpiperazinsulfonyl)-amino-3-phenylmethyl-2-oxo-1-piperidinyl)-3-(4-thiazolyl)-propionyl]-(2S,3R,4S)-1-cyclohexyl-3,4-dihydroxy-6(2-pyridyl)-2-hexylamin

Die Titelverbindung wird aus (2S)-((3S)-(N-Methylpiperazinsulfonyl)-amino-3-phenylmethyl-2-oxo-1-piperidinyl)-3-(4-thiazolyl)-propionsäure (bekannt aus EP-A 365 992, Beispiel 151) und der Verbindung aus Beispiel 1a nach dem Verfahren aus Beispiel 1b dargestellt.
MS(FAB): 796 (M + H)

Beispiel 11

N-[(2S)-3-(Imidazol-4-yl)-3-((3S)-(4-methylphenylsulfonyl)-amino)-3-phenylmethyl-2-oxo-1-piperidinyl)-propionyl]-(2S,3R,4S)-1-cyclohexyl-3,4-dihydroxy-6(2-pyridyl)-2-hexylamin

Die Titelverbindung wird aus (2S)-2-((1-tert.-Butyloxycarbonyl)-imidazol-4-yl-methyl)-3-((3S)-(4-methylphenylsulfonyl)amino)-3-phenylmethyl-2-oxo-1-piperidinyl)essigsäure (bekannt aus EP-A 365 992, Beispiel 155) und der Verbindung aus Beispiel 1a nach dem Verfahren des Beispiels 1b und anschließender Schutzgruppenabspaltung in THF/Essigsäure/Wasser hergestellt.
MS(FAB): 771 (M + H).

## Patentansprüche

1. Verbindung der Formel (I)

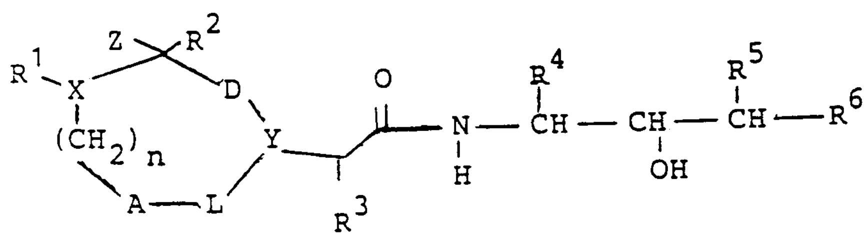

(I)

in welcher bedeuten:

X N, O oder CH;

$R^1$ nicht vorhanden, Wasserstoff, eine N-Schutzgruppe, Aryl, heterocyclisch oder $R^7$-Q mit

1) $R^7$ gleich kurzkettiges Alkyl, Amino, Alkylamino, Dialkylamino, (Alkoxyalkyl)(alkyl)amino, (Alkoxyalkoxyalkyl)(alkyl)amino, Aryl, Arylalkyl, Aminoalkyl, N-geschütztes Aminoalkyl, Alkoxy, substituiertes kurzkettiges Alkyl mit einem Substituenten aus der Gruppe: Alkoxy, Thioalkoxy, Halogen, Alkylamino, N-geschütztes (Alkyl)amino und Dialkylamino,

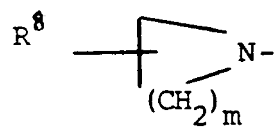

mit m gleich 1-5 und $R^8$ Wasserstoff, Hydroxy, Alkoxy, Thioalkoxy, Alkoxyalkoxy, Polyalkoxy, Amino, N-geschütztes Amino, Alkylamino, N-geschütztes Alkylamino oder Dialkylamino,

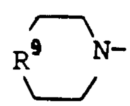

mit $R^9$ gleich O, S, $SO_2$, O=C oder $R^{10}N$ mit $R^{10}$ gleich Wasserstoff, kurzkettiges Alkyl oder eine N-Schutzgruppe,

2) Q ist C=O oder $CH_2$,

wobei X gleich N ist, wenn $R^1$ eine N-Schutzgruppe ist,

$R^{11}S(O)_2$ mit $R^{11}$ gleich Amino, Alkylamino, Dialkylamino, kurzkettiges Alkyl, Halogenalkyl, Aryl, p-Biphenyl, heterocyclisch oder $(R^{12})_2P)O)$ mit $R^{12}$ gleich Alkoxy, Alkylamino oder Dialkylamino,

A und L unabhängig voneinander entweder nicht vorhanden, C=O, $SO_2$ oder $CH_2$;

D C=O, $SO_2$ oder $CH_2$,

Y N oder CH

$R^2$ Wasserstoff, kurzkettiges Alkyl,, Cycloalkylalkyl, $-CH_2-R^{13}-(CH_2)_q-R^{14}$, wobei q gleich 0, 1 oder 2 ist,

$R^{13}$ nicht vorhanden oder O, NH oder S bei q gleich 1 oder 2 ist;

$R^{14}$ für Alkyl oder einen Heterocyclus steht;

Z Wasserstoff oder $-R^{15}C(O)R^{16}$, $-R^{15}S(O)_2R^{16}$ oder $-R^{15}-C(S)R^{16}$,wobei

$R^{15}$ gleich NH, $-N(R^{17})-$ mit $R^{17}$ gleich kurzkettiges Alkyl oder Benzyl, $CH_2$ und

$R^{16}$ ist Alkoxy, Benzyloxy, Alkylamino, Dialkylamino, Aryl oder heterocyclisch;

$R^3$ Wasserstoff, kurzkettiges Alkyl, kurzkettiges Alkenyl, Cycloalkylalkyl, Cycloalkenylalkyl, Alkoxyalkyl, Thioalkoxyalkyl, (Alkoxyalkoxy)alkyl, (Polyalkoxy)alkyl, Arylalkyl oder heterocyclisch substituiertes Alkyl;

n 0 oder 1

$R^4$ Wasserstoff, $(C_1-C_{10})$-Alkyl, $(C_3-C_6)$-Cycloalkyl, $(C_3-C_8)$-Cycloalkyl-$(C_1-C_4)$-alkyl, $(C_6-C_{12})$-Aryl oder $(C_6-C_{12})$-Aryl-$(C_1-C_4)$-alkyl

$R^5$ Wasserstoff, $(C_1-C_{10})$-Alkyl, $(C_6-C_{12})$-Aryl, $(C_6-C_{12})$-Aryl-$(C_1-C_4)$-alkyl, Hydroxy oder Amino;

$R^6$ einen Rest der Formel (II)

$(CH_2)_s-CHR^{18}-Het$ (II)

wobei

$R^{18}$ für Wasserstoff, $(C_1-C_7)$-Alkyl, $(C_1-C_4)$-Alkoxy, $(C_1-C_4)$-Alkylthio, $(C_1-C_4)$-Alkylamino, Hydroxy, Azido oder Halogen steht, und Het für einen 5- bis 7-gliedrigen heterocyclischen Ring steht, der benzanneliert, aromatisch, teilhydriert oder vollständig hydriert sein kann; der als Heteroatome einen oder zwei gleiche oder verschiedene Reste aus der Gruppe N, O, S, NO, SO, $SO_2$ enthalten kann und der durch einen oder zwei gleiche oder verschiedene Reste aus

der Reihe ($C_1$-$C_4$)-Alkyl, Allyl, ($C_1$-$C_4$)-Alkoxy, Hydroxy, Halogen, Amino, Mono- oder Di-($C_1$-$C_4$)-alkylamino und $CF_3$ substituiert sein kann; und

s 0, 1, 2, 3 oder 4 bedeutet;

sowie deren physiologisch verträglichen Salze.

**2.** Verbindung der Formel (I) gemäß Anspruch 1, dadurch gekennzeichnet, daß die Symbole folgende Bedeutungen haben:

$R^4$ Isobutyl, Benzyl oder Cyclohexylmethyl;

$R^5$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy;

$R^6$ einen Rest der Formel (II), worin $R^{18}$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido oder Halogen steht,

s 0, 1 oder 2;

X N oder CH,

$R^1$ nicht vorhanden, Wasserstoff, Benzyl, 2-Pyridyl, Piperazin-1-yl, (4-OMe)phenyl, tert.-Butyloxycarbonyl, Isobutyl, Benzyloxycarbonyl, $Me_2NC(O)$-, 4-Methyl-piperazin-1-yl-carbonyl, Morpholin-1-yl-carbonyl, p-Toluolsulfonyl, oder 4-Methyl-piperazin-1-yl-sulfonyl;

A und L unabhängig voneinander entweder nicht vorhanden oder $CH_2$,

D C = O oder $CH_2$;

Y gleich N

$R^2$ Wasserstoff oder Benzyl;

Z Wasserstoff, BOC-NH, CbZ-NH, p-Toluolsulfonylamino, 4-Methylpiperazin-1-yl-sulfonylamino, 4-Methylpiperazin-1-yl, N-Methyl-N-(4-methyl-piperazin-1-yl-sulfonyl)-amino;

$R^3$ Wasserstoff, Methyl, n-Butyl, 4-Imidazomethyl, 4-Thiazolylmethyl oder N-BOC-4-(imidazomethyl);

n Null

und die übrigen Reste wie in Anspruch 1 definiert sind, sowie deren pyhsiologisch verträglichen Salze.

**3.** Verbindung der Formel (I) gemäß Anspruch 1 oder 2, dadurch gekennzeichnet, daß die Symbole folgende Bedeutungen haben:

$R^4$ Isobutyl, Benzyl oder Cyclohexylmethyl;

$R^5$ Wasserstoff oder Hydroxy;

$R^6$ einen Rest der Formel (II), worin

$R^{18}$ für Wasserstoff oder Fluor steht,

Het für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Alkyl, Fluor, Chlor, Brom, $CF_3$ und Methoxy substituiert sein können, und

s 0, 1 oder 2;

und die übrigen Reste und Variablen wie in Anspruch 1 oder 2 definiert sind, sowie deren physiologisch verträglichen Salze.

**4.** Verfahren zur Herstellung einer Verbindung der Formel (I) gemäß Anspruch 1, wobei man ein Fragment der Formel (III) mit endständiger Carboxylgruppe oder dessen reaktives Derivat mit einem Fragment der Formel (IV) mit freier Aminogruppe kuppelt,

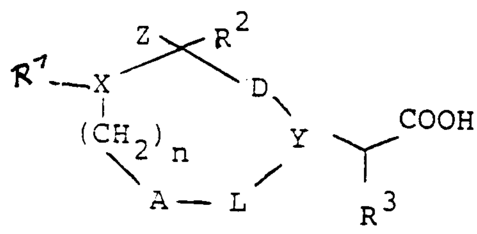

(III)

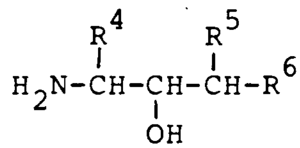

(IV)

gegebenenfalls zum Schutz weiterer funktioneller Gruppen (eine) temporär eingeführte Schutzgruppe(n) abspaltet und die so erhaltene Verbindung gegebenenfalls in ein physiologisch verträgliches Salz überführt.

**5.** Verbindung der Formel (I) gemäß einem der Ansprüche 1 bis 3 zur Anwendung als Heilmittel.

**6.** Verbindung der Formel (I) gemäß einem der Ansprüche 1 bis 3 zur Anwendung als Heilmittel bei der Behandlung des Bluthochdrucks.

**7.** Pharmazeutische Zubereitung enthaltend einen Wirkstoff und gegebenenfalls Trägerstoffe und weitere Zusätze, dadurch gekennzeichnet, daß sie als Wirkstoff mindestens eine Verbindung der Formel (I) gemäß Anspruch 1 enthält.

**8.** Verfahren zur Herstellung einer pharmazeutischen Zubereitung gemäß Anspruch 7, dadurch gekennzeichnet, daß man den oder die Wirkstoffe zusammen mit einem physiologisch unbedenklichen Träger und gegebenenfalls weiteren Zusätzen in eine geeignete Darreichungsform bringt.

**Patentansprüche für folgende Vertragsstaaten : ES, GR**

**1.** Verfahren zur Herstellung einer Verbindung der Formel (I)

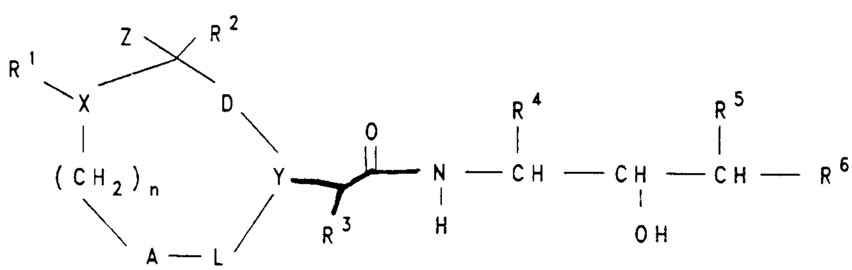

( I )

in welcher bedeuten:

X N, O oder CH;

$R^1$ nicht vorhanden, Wasserstoff, eine N-Schutzgruppe, Aryl, heterocyclisch oder $R^7$-Q mit

1) $R^7$ gleich kurzkettiges Alkyl, Amino, Alkylamino, Dialkylamino, (Alkoxyalkyl)(alkyl)amino, (Alkoxyalkoxyalkyl)(alkyl)amino, Aryl, Arylalkyl, Aminoalkyl, N-geschütztes Aminoalkyl, Alkoxy, substituiertes kurzkettiges Alkyl mit einem Substituenten aus der Gruppe: Alkoxy, Thioalkoxy, Halogen, Alkylamino, N-geschütztes (Alkyl)amino und Dialkylamino,

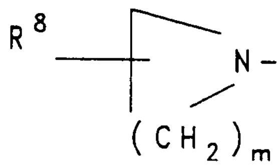

mit m gleich 1-5 und $R^8$ Wasserstoff, Hydroxy, Alkoxy, Thioalkoxy, Alkoxyalkoxy, Polyalkoxy, Amino, N-geschütztes Amino, Alkylamino, N-geschütztes Alkylamino oder Dialkylamino,

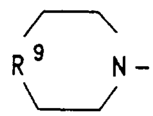

mit $R^9$ gleich O, S, $SO_2$, O=C oder $R^{10}N$ mit $R^{10}$ gleich Wasserstoff, kurzkettiges Alkyl oder eine N-Schutzgruppe,
2) Q ist C=O oder $CH_2$,
wobei X gleich N ist, wenn $R^1$ eine N-Schutzgruppe ist,

$R^{11}S(O)_2$ mit $R^{11}$ gleich Amino, Alkylamino, Dialkylamino, kurzkettiges Alkyl, Halogenalkyl, Aryl, p-Biphenyl, heterocyclisch oder $(R^{12})_2P)O)$ mit $R^{12}$ gleich Alkoxy, Alkylamino oder Dialkylamino,

A und L unabhängig voneinander entweder nicht vorhanden, C=O, $SO_2$ oder $CH_2$;

D C=O, $SO_2$ oder $CH_2$,

Y N oder CH

$R^2$ Wasserstoff, kurzkettiges Alkyl,, Cycloalkylalkyl, $-CH_2-R^{13}-(CH_2)_q-R^{14}$, wobei q gleich 0, 1 oder 2 ist,
$R^{13}$ nicht vorhanden oder O, NH oder S bei q gleich 1 oder 2 ist;
$R^{14}$ für Alkyl oder einen Heterocyclus steht;

Z Wasserstoff oder $-R^{15}C(O)R^{16}$, $-R^{15}S(O)_2R^{16}$ oder $-R^{15}-C(S)R^{16}$,wobei
$R^{15}$ gleich NH, $-N(R^{17})-$ mit $R^{17}$ gleich kurzkettiges Alkyl oder Benzyl, $CH_2$ und
$R^{16}$ ist Alkoxy, Benzyloxy, Alkylamino, Dialkylamino, Aryl oder heterocyclisch;

$R^3$ Wasserstoff, kurzkettiges Alkyl, kurzkettiges Alkenyl, Cycloalkylalkyl, Cycloalkenylalkyl, Alkoxyalkyl, Thioalkoxyalkyl, (Alkoxyalkoxy)alkyl, (Polyalkoxy)alkyl, Arylalkyl oder heterocyclisch substituiertes Alkyl;

n 0 oder 1

$R^4$ Wasserstoff, $(C_1-C_{10})$-Alkyl, $(C_3-C_6)$-Cycloalkyl, $(C_3-C_8)$-Cycloalkyl-$(C_1-C_4)$alkyl, $(C_6-C_{12})$-Aryl oder $(C_6-C_{12})$-Aryl-$(C_1-C_4)$-alkyl

$R^5$ Wasserstoff, $(C_1-C_{10})$-Alkyl, $(C_6-C_{12})$-Aryl, $(C_6-C_{12})$-Aryl-$(C_1-C_4)$-alkyl, Hydroxy oder Amino;

$R^6$ einen Rest der Formel (II)

$(CH_2)_s-CHR^{18}-Het$ (II)

wobei
$R^{18}$ für Wasserstoff, $(C_1-C_7)$-Alkyl, $(C_1-C_4)$-Alkoxy, $(C_1-C_4)$-Alkylthio, $(C_1-C_4)$-Alkylamino, Hydroxy, Azido oder Halogen steht, und Het für einen 5- bis 7-gliedrigen heterocyclischen Ring steht, der benzanneliert, aromatisch, teilhydriert oder vollständig hydriert sein kann; der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Reihe $(C_1-C_4)$-Alkyl, Allyl, $(C_1-C_4)$-Alkoxy, Hydroxy, Halogen, Amino, Mono- oder Di-$(C_1-C_4)$-alkylamino und $CF_3$ substituiert sein kann; und

s 0, 1, 2, 3 oder 4 bedeutet;

sowie deren physiologisch verträglichen Salze,
wobei man ein Fragment der Formel (III) mit endständiger Carboxylgruppe oder dessen reaktives Derivat mit einem Fragment der Formel (IV) mit freier Aminogruppe kuppelt,

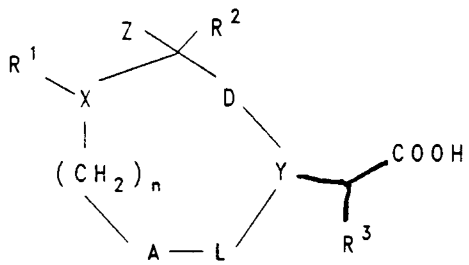

(III)

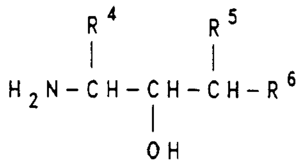

(IV)

gegebenenfalls zum Schutz weiterer funktioneller Gruppen (eine) temporär eingeführte Schutzgruppe(n) abspaltet und die so erhaltene Verbindung gegebenenfalls in ein physiologisch verträgliches Salz überführt.

**2.** Verfahren gemäß Anspruch 1, dadurch gekennzeichnet, daß die Symbole folgende Bedeutungen haben:

$R^4$ Isobutyl, Benzyl oder Cyclohexylmethyl;
$R^5$ Wasserstoff, $(C_1\text{-}C_5)$-Alkyl, $(C_6\text{-}C_{10})$-Aryl, $(C_6\text{-}C_{10})$-Aryl-$(C_1\text{-}C_4)$-alkyl oder Hydroxy;
$R^6$ einen Rest der Formel (II), worin $R^{18}$ für Wasserstoff, $(C_1\text{-}C_4)$-Alkyl, $(C_1\text{-}C_4)$-Alkoxy, $(C_1\text{-}C_4)$-Alkylthio, $(C_1\text{-}C_4)$-Alkylamino, Hydroxy, Azido oder Halogen steht,
s 0, 1 oder 2;
X N oder CH,
$R^1$ nicht vorhanden, Wasserstoff, Benzyl, 2-Pyridyl, Piperazin-1-yl, (4-OMe)phenyl, tert.-Butyloxycarbonyl, Isobutyl, Benzyloxycarbonyl, $Me_2NC(O)$-, 4-Methyl-piperazin-1-yl-carbonyl, Morpholin-1-yl-carbonyl, p-Toluolsulfonyl, oder 4-Methyl-piperazin-1-yl-sulfonyl;
A und L unabhängig voneinander entweder nicht vorhanden oder $CH_2$,
D C=O oder $CH_2$;
Y gleich N
$R^2$ Wasserstoff oder Benzyl;
Z Wasserstoff, BOC-NH, CbZ-NH, p-Toluolsulfonylamino, 4-Methylpiperazin-1-yl-sulfonylamino, 4-Methylpiperazin-1-yl, N-Methyl-N-(4-methyl-piperazin-1-yl-sulfonyl)-amino;
$R^3$ Wasserstoff, Methyl, n-Butyl, 4-Imidazomethyl, 4-Thiazolylmethyl oder N-BOC-4-(imidazomethyl);
n Null

und die übrigen Reste wie in Anspruch 1 definiert sind, sowie deren pyhsiologisch verträglichen Salze.

**3.** Verfahren gemäß Anspruch 1, dadurch gekennzeichnet, daß die Symbole folgende Bedeutungen haben:

$R^4$ Isobutyl, Benzyl oder Cyclohexylmethyl;
$R^5$ Wasserstoff oder Hydroxy;
$R^6$ einen Rest der Formel (II), worin
$R^{18}$ für Wasserstoff oder Fluor steht,
Het für einen 2-, 3- oder 4-Pyridinrest, einen 2-, 4- oder 5-Imidazolrest oder einen 2-Oxazolinrest steht, wobei die genannten Heterocyclen jeweils durch einen oder zwei gleiche oder verschiedene Reste aus der Reihe Methyl, Ethyl, Propyl, Alkyl, Fluor, Chlor, Brom, $CF_3$ und Methoxy substituiert sein können, und
s 0, 1 oder 2;

und die übrigen Reste und Variablen wie in Anspruch 1 definiert sind, sowie deren physiologisch verträglichen Salze.

**4.** Verfahren zur Herstellung einer pharmazeutischen Zubereitung enthaltend eine Verbindung herstellbar gemäß Anspruch 1, dadurch gekennzeichnet, daß man die Verbindung zusammen mit einem physiologisch unbedenklichen Träger und gegebenenfalls weiteren Zusätzen in eine geeignete Darreichungsform bringt.

Europäisches Patentamt

# EUROPÄISCHER RECHERCHENBERICHT

Nummer der Anmeldung

EP 92 11 0244

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 365 992 (ABBOTT LABORATORIES)<br>* Verbindungen mit 1; Seiten 65-73 *<br>--- | 1,5 | C 07 D 295/20<br>C 07 D 401/12<br>C 07 D 401/14<br>C 07 D 413/14<br>C 07 D 417/14<br>A 61 K 31/535<br>C 07 K 5/06 |
| Y | EP-A-0 283 970 (FUJISAWA PHARMACEUTICAL CO.)<br>* Beispiele 1,2 *<br>--- | 1,5 | |
| Y | EP-A-0 229 667 (ABBOTT LABORATORIES)<br>* Beispiele 24,29,118 *<br>--- | 1,5 | |
| Y,D | EP-A-0 370 454 (HOECHST AG)<br>* Beispiele *<br>--- | 1,5 | |
| Y | EP-A-0 394 853 (HOECHST AG)<br>* Beispiele *<br>--- | 1,5 | |
| Y,D | EP-A-0 373 497 (HOECHST AG)<br>* Beispiel 45 *<br>--- | 1,5 | |
| A | EP-A-0 309 766 (BANYU PHARMACEUTICAL CO. LTD.)<br>* Seite 17; Beispiele *<br>--- | 1,5 | **RECHERCHIERTE SACHGEBIETE (Int. Cl.5)** |
| A | EP-A-0 307 837 (ABBOTT LABORATORIES)<br>* Beispiele 106,108,115,152 *<br>----- | 1,5 | C 07 D<br>A 61 K<br>C 07 K |

Der vorliegende Recherchenbericht wurde für alle Patentansprüche erstellt

| Recherchenort | Abschlußdatum der Recherche | Prüfer |
|---|---|---|
| BERLIN | 14-09-1992 | FRELON D.L.M.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)